# FASCICULE DE BREVET EUROPEEN

(11) **EP 1 409 008 B1**
(45) Date de publication et mention de la délivrance du brevet: **19.01.2011**
(21) Numéro de dépôt: 02774845.8
(22) Date de dépôt: 26.07.2002
(51) Int. Cl.: A61K 38/48, A61K 38/17, A61K 48/00, A61P 35/00, A61P 29/00, A61P 17/06, A61P 9/10, A61P 27/00

(54) **UTILISATION D'UNE MOLÉCULE D'ACIDE NUCLÉIQUE CODANT LE DOMAINE DISINTEGRINE D'UNE ADAMALYSINE COMME AGENT ANTI-ANGIOGENIQUE, ANTI-INVASIF ET ANTI-METASTATIQUE**
VERWENDUNG EINES NUKLEINSÄUREMOLEKÜLS KODIEREND FÜR DAS DISINTEGRIN-DOMAINS EINES ADAMALYSINS ALS ANTI-ANGIOGENESIS, ANTI-INVASIONS UND ANTI-METASTASIS MITTEL
USE OF A NUCLEIC ACID MOLECULE CODING THE DISINTEGRIN DOMAIN OF AN ADAMALYSIN AS ANTI-ANGIOGENIC, ANTI-INVASIVE AND ANTI-METASTATIC AGENT

(30) Priorité: 26.07.2001 FR 0110015
(43) Date de publication de la demande: 21.04.2004
(73) Titulaire: Institut National de la Santé et de la Recherche Médicale (INSERM), 75654 Paris Cedex 13 (FR)
(72) Inventeur: TROCHON, Véronique, F-75014 Paris (FR); LU, He, F-93800 Epinay-sur-Seine (FR); SORIA, Claudine, F-95150 Taverny (FR)
(74) Mandataire: Jacobson, Claude
(86) Numéro de dépôt international: PCT/FR2002/002691
(87) Numéro de publication internationale: WO 2003/009866

(56) Documents cités:
- EP-A1- 1 803 810
- J. KRÄTZSCHMAR ET AL.: "Metargidin, a membrane-anchored metalloprotease-disintegrin protein with an RGD integrin binding sequence." THE JOURNAL OF BIOLOGICAL CHEMISTRY, vol. 271, no. 9, 1 mars 1996 (1996-03-01), pages 4593-4596, XP000644305 BALTIMORE, MD, US cité dans la demande
- X.-P. ZHANG ET AL.: "Specific interaction of the recombinant disintegrin-like domain of MDC-15 (metargidin, ADAM-15) with integrin alphaV beta3." JOURNAL OF BIOLOGICAL CHEMISTRY, vol. 273, no. 13, 27 mars 1998 (1998-03-27), pages 7345-7350, XP002201315 BALTIMORE, MD, US cité dans la demande
- B. HERREN ET AL.: "Expression of a disintegrin-like protein in cultured human vascular cells and in vivo." FASEB JOURNAL FOR EXPERIMENTAL BIOLOGY, vol. 11, février 1997 (1997-02), pages 173-180, XP002201316 BETHESDA, MD, US cité dans la demande
- C.H. YEH ET AL.: "Accutin, a new disintegrin, inhibits angiogenesis in vitro and in vivo by acting as integrin alphaV beta3 antagonist and inducing apoptosis." BLOOD., vol. 92, no. 9, 1 novembre 1998 (1998-11-01), pages 3268-3276, XP002201317 W.B.SAUNDERS COMPAGNY, ORLANDO, FL., US ISSN: 0006-4971 cité dans la demande
- YEH C H ET AL: "Accutin, a new disintegrin, inhibits angiogenesis in vitro and in vivo by acting as integrin alphaV beta3 antagonist and inducing apoptosis" BLOOD, AMERICAN SOCIETY OF HEMATOLOGY, US, vol. 92, no. 9, 1 November 1998 (1998-11-01), pages 3268-3276, XP002201317 ISSN: 0006-4971

## Description

La présente invention concerne le domaine de l'inhibition des événements qui concourent au processus angiogénique, invasif et métastatique, lequel est impliqué dans de nombreuses pathologies, comme le cancer, les maladies inflammatoires, l'athérosclérose, l'angiogenèse pathologique de la rétine.

L'invention est basée sur la mise en évidence des fonctions anti-angiogéniques, anti-invasives et antimétastatiques d'un fragment d'une molécule présente sur les cellules endothéliales humaines. L'invention concerne l'utilisation d'une molécule d'acide nucléique comprenant ou constituée d'une séquence polynucléotidique codant le domaine disintégrine de la métargidine (Krätzschmar et coll., 1996) aussi désigné ci-après « AMEP » pour « antiangiogenic metargidin peptide ».

Ce fragment présente l'originalité, par rapport aux autres substances antiangiogéniques qui ont été décrites dans la littérature :
- d'inhiber simultanément toutes les étapes de l'angiogénèse : la migration et la prolifération des cellules endothéliales, leur adhésion à différents substrats de la matrice, la formation de structures de type capillaire, et
- d'induire une apoptose des cellules endothéliales.

De plus, de manière inattendue, ce fragment présente la capacité d'une part d'inhiber l'invasion de cellules cancéreuses et d'autre part d'empêcher la formation de métastases, notamment, de cellules qui expriment à leur surface l'intégrine alpha v bêta 3

L'angiogénèse désigne un procédé morphogénétique par lequel de nouveaux capillaires sanguins sont formés par bourgeonnement de vaisseaux existant en réponse à une stimulation. Lors de l'angiogénèse *in vivo*, les néocapillaires prennent naissance à partir des capillaires ou des veinules post-capillaires, jamais des artères, artérioles ou des veines. Ainsi, un facteur angiogénique est une molécule qui permet l'initiation et/ou le maintien de l'angiogénèse, comme par exemple le FGF2. Un facteur antiangiogénique est donc une molécule qui inhibe l'angiogénèse en visant une ou plusieurs étapes clef de l'angiogénèse.

L'adhésion consiste dans la capacité des cellules de s'attacher à une matrice extracellulaire. Ce phénomène fait intervenir de nombreuses molécules d'adhésion présentes à la surface des cellules.

La migration des cellules fait intervenir des enzymes qui permettent aux cellules de dégrader les composés de la matrice, ainsi que des molécules d'adhésion qui assurent l'ancrage des cellules à la matrice. De plus, l'architecture dynamique du cytosquelette permet aux cellules d'alterner les périodes d'adhésion et de décollement indispensable à la motilité.

La prolifération est un phénomène qui se rapporte à la division des cellules au cours du temps.

L'apoptose consiste dans la capacité intrinsèque des cellules normales de déclencher leur propre suicide selon un programme complexe appelé mort cellulaire. L'anoikis est une forme de l'apoptose induite dans les cellules normales résultant d'une perte de leur adhésion au substrat.

L'invasion est une multiplication exagérée d'une classe d'éléments anatomiques qui entraîne la substitution par ceux-ci des éléments adjacents.

La métastase est un foyer de cellules cancéreuses, en rapport avec un cancer préexistant, dit « primitif », mais développé à distance de ce dernier et sans continuité avec lui. La dissémination de ces foyers secondaires se fait par voie lymphatique ou sanguine.

Le développement d'une tumeur et sa dissémination dans divers organes dépendent de la vascularisation intra et périvasculaire aussi appelée angiogénèse (Folkman, 1984). Le ciblage du processus angiogénique est une approche thérapeutique nouvelle et représente une révolution dans le traitement des cancers. Ainsi, de nombreuses molécules antiangiogéniques sont actuellement en cours d'essais cliniques.

La vitaxine est anticorps anti-intégrine alpha v bêta 3 humanisé qui induit une inhibition de la prolifération des cellules endothéliales ainsi qu'un effet proapoptotique (Brooks et coll., 1994; Hammes et coll., 1996), mais elle ne modifie pas leur migration. L'intégrine alpha v bêta 3 est une molécule d'adhésion exprimée préférentiellement par les cellules endothéliales des néovaisseaux et certaines cellules cancéreuses. Elle interagit avec certains composés de la matrice extracellulaire notamment la vitronectine, la fibronectine, la laminine, le collagène IV et la fibrine induisant l'adhésion et la migration des cellules endothéliales. Le rôle majeur de l'intégrine alpha v bêta 3 dans l'angiogénèse a été largement décrit (revue Eliceiri et Cheresh, 2000).

Le marimastat bloque l'activité protéolytique des métalloprotéinases et par conséquent inhibe la migration des cellules endothéliales mais n'a aucun effet sur la prolifération de ces dernières. Les métalloprotéinases (MMPs) appartiennent en effet à la grande famille des enzymes qui permettent de dégrader l'ensemble des composés de la matrice extracellulaire, indispensables à la migration des cellules endothéliales. D'autres enzymes, appartenant aux sérines protéases, participent aussi à la migration cellulaire, comme l'activateur du plasminogène de type urokinase (uPA) lorsqu'il est lié à son récepteur ancré à la surface membranaire (u-PAR) et la plasmine.

L'ensemble de ces médicaments ont pour but de bloquer l'angiogénèse, mais leur action se limite à une ou deux étapes de ce processus contrairement à l'AMEP qui est pluripotent. Ainsi, l'AMEP bloque non seulement l'ensemble des fonctions angiogéniques de l'intégrine alpha v bêta 3, contre laquelle elle est dirigée initialement, à savoir la prolifération et l'adhésion des cellules endothéliales, mais, de manière surprenante, elle induit également une inhibition complète de la migration de ces cellules ainsi qu'une inhibition de la formation de structures de type capillaire. Son effet proapoptotique sur ces cellules, indépendant d'une modification de leur cycle cellulaire, en fait également son originalité.

De plus, de manière inattendue, l'AMEP possède à la fois des capacités anti-invasives et antimétastatiques importantes.

Les adamalysines, aussi nommées ADAM, pour « a disintegrin and metalloprotein » ou MDC pour « metalloprotein-rich, disintegrin-rich and cystein-rich protein », sont une famille de protéines ancrées dans la membrane plasmique des cellules. La structure commune à l'ensemble des 29 adamalysines décrites se compose :
- d'un domaine métalloprotéinase dont l'activité catalytique protéasique dépend du zinc,
- un domaine disintégrine, et
- un domaine riche en cystéine et en répétition de type EGF (Wolfsberg et coll., 1995).

Il convient cependant de remarquer que sur l'ensemble des adamalysines, seulement une dizaine ont un domaine métalloprotéinase possédant une activité catalytique. Le rôle physiologique des différentes adamalysines est extrêmement varié : régulation de l'adhérence cellulaire, libération d'un ligand, activation d'un récepteur, fusion de cellules (revue, Primakoff et Myles, 2000). Le mode d'action de ces molécules reste cependant inconnu.

L'AMEP est à différencier des disintégrines de serpent qui ont été décrits dans la littérature sous deux aspects :
- Les disintégrines de serpent exercent une action limitée sur les différentes étapes de l'angiogenèse. L'accutine (Yeh et coll., 1998) par exemple inhibe l'adhésion des cellules endothéliales à différents composés de la matrice et induit leur apoptose tandis que la Salmosine (Kang et coll., 1999) induit une inhibition de l'adhésion des cellules endothéliales et de leur prolifération induite par le FGF2.
- L'AMEP présente également l'avantage d'être d'origine humaine et par conséquent, elle n'a pas le caractère antigénique des disintégrines de serpent qui présente un caractère immunogène les empêchant d'être utilisées comme médicaments dans un traitement à long terme requis en thérapie anticancéreuse.

L'invention a donc pour objet, une molécule d'acide nucléique comprenant ou constituée d'une séquence polynucléotidique codant le domaine disintégrine de la Métargidine ou un dérivé de celui-ci comme agent anti-angiogénique, anti-invansif eou anti-mitastatique. L'invention s'intéresse donc à l'utilisation d'une substance protéique comprenant ou constituée du domaine disintégrine de la métargidine dont la séquence en acides aminés est représentée dans la liste de séquence en annexe sous le numéro SEQ ID NO :2 ou un dérivé de celle-ci.

Le fragment AMEP est remarquable en ce qu'il est capable d'inhiber l'invasion tumorale, la formation de métastases et toutes les étapes de l'angiogénèse, c'est-à-dire à la fois la migration et la prolifération des cellules endothéliales contrairement à ce qui avait été supposé par certains auteurs (Zhang et coll., 1998) qui considérait que ce domaine disintégrine en se liant à l'intégrine alpha v bêta 3 pourrait être uniquement impliqué dans l'agrégation homotypique des cellules endothéliales durant l'angiogenèse. En outre, l'action inhibitrice de l'AMEP sur l'angiogénèse s'observe en absence de toute addition de facteurs angiogéniques.

La nécessité d'utiliser des facteurs angiogéniques exogènes s'observe par contre pour mettre en évidence l'effet antiangiogénique des anticorps anti-alpha v bêta 3. Ils inhibent l'angiogénèse uniquement après induction par le FGF2, un facteur angiogénique indispensable au maintien de l'angiogénèse (Klein et coll., 1993). L'intérêt thérapeutique de l'AMEP par rapport à d'autres peptides possédant la séquence RGD qui sont décrits comme de simples inhibiteurs de l'adhésion des cellules endothéliales (Kostetsky et Artemjev, 2000), réside également dans son spectre d'action.

L'invention concerne des séquences polynucléotidiques codant le domaine disintégrine de la métargidine et les dérivés de celui-ci. De tels dérivés constituent des équivalents fonctionnels présentant des propriétés anti-angiogéniques, anti-invasives et anti-métastatiques, que l'homme du métier est à même de déterminer à partir de l'enseignement de la présente invention et plus particulièrement des modèles et des tests rapportés dans la partie expérimentale ci-après. Il s'agit de séquences modifiées par délétion, addition, suppression ou remplacement d'un acide aminé. ces dérivés.

L'invention s'intéresse donc également tout spécialement à une molécule d'acide nucléique comprenant une séquence polynucléotidique codant le domaine disintégrine de la métargidine dont la séquence en est représentée dans la liste de séquence en annexe sous le numéro SEQ ID NO :1 ou un dérivé de celle-ci. La séquence codante de ce domaine est constituée de 276 nucléotides (Met-420 à Glu-511).

Ladite séquence est avantageusement placée sous le contrôle de séquences de régulation de son expression. Une telle molécule d'acide nucléique est par exemple un vecteur, comme :
- un plasmide d'expression codant pour le fragment anti-angiogénique AMER ou un dérivé quelle que soit la technique de transfert,
- un plasmide d'expression, codant pour une protéine de fusion entre ledit fragment ou un dérivé et un domaine protéique facilitant la purification (plasmide type pGEX) ou facilitant le ciblage tissulaire,
- un plasmide ou autre type de vecteur d'expression codant pour ledit fragment AMEP ou un dérivé, spécifique d'un organisme hôte autre qu'une bactérie, par exemple un baculovirus dans une cellule d'insecte ou un plasmide dans une cellule eucaryote.

Une telle molécule d'acide nucléique peut être utilisée dans des protocoles de thérapie génique ou de thérapie cellulaire consistant à administrer ladite molécule ou des cellules transformées par ladite molécule a un individu de façon à exprimer tout ou partie du domaine disintégrine au niveau d'un site à traiter.

Une telle molécule d'acide nucléique est aussi utile pour préparer la substance protéique de l'invention. Ainsi, l'AMEP humaine a été synthétisé à partir de bactéries et cellules eucaryotes transformées avec un plasmide codant pour l'AMEP. Plus précisément *Escherichia Coli* (clone DH5 alpha) a été utilisé comme système de production bactérien et le muscle *tibia cranial* comme système de production eucaryote, mais la levure ou tout autre système de production pourrait être utilisé.

La mise en évidence de l'action inhibitrice de l'AMEP sur toutes les étapes de l'angiogénèse (migration, prolifération, adhésion, apoptose des cellules endothéliales et la formation de structures de type capillaire) et sur l'invasion tumorale et la formation de métastases, permet d'offrir un nouveau médicament antitumoral dans le traitement des cancers. En effet, contrairement aux autres inhibiteurs de l'angiogénèse décrit dans la littérature jusqu'à maintenant, l'AMEP exerce une activité anti-invasive, anti-métastatique et anti-angiogénique intrinsèque, pluripotente et exceptionnelle. Il inhibe à la fois la migration et la prolifération des cellules endothéliales de différentes origines (macrovasculaire ou microvasculaire, transformées ou non) ainsi que l'adhésion des cellules (sur fibrinogène, vitronectine et fibronectine) et la formation de structures de type capillaire dans des modèles. tridimensionnels *in vitro.* Un effet proapoptotique de L'AMEP est également mis en évidence. *In vivo,* l'AMEP bloque la croissance tumorale en inhibant la formation de vaisseaux sanguins et la dissémination métastatique, en particulier, de cellules exprimant l'intégrine alpha V bêta 3.

La présente invention vise donc à offrir un nouvelle agent pour le traitement et/ou la prévention des pathologies cancéreuses en général ainsi que des maladies où l'angiogenèse contribue à la pathogénie des affections comme les maladies inflammatoires, le psoriasis, l'athérosclérose, la dégénérescence maculaire etc.

L'agent actif est associé dans les médicaments selon l'invention avec tout véhicule pharmaceutiquement acceptable connu de l'homme du métier adapté au mode d'administration utilisé. Ainsi, les médicaments selon l'invention peuvent être administrés par :
- seul, par voie systémique, locale, orale ou en implant
- par thérapie cellulaire ou génique
- en association avec d'autres principes actifs
- sous quelque forme galénique que ce soit, telle qu'une forme de nanoparticules par exemple

D'autres avantages et caractéristiques de l'invention apparaîtront de la description ci-après concernant la préparation de l'AMEP et son activité anti-angiogénique, anti-invasive et anti-métastatique *in vitro* et *in vivo*, et où il sera fait référence aux dessins en annexe dans lesquels :

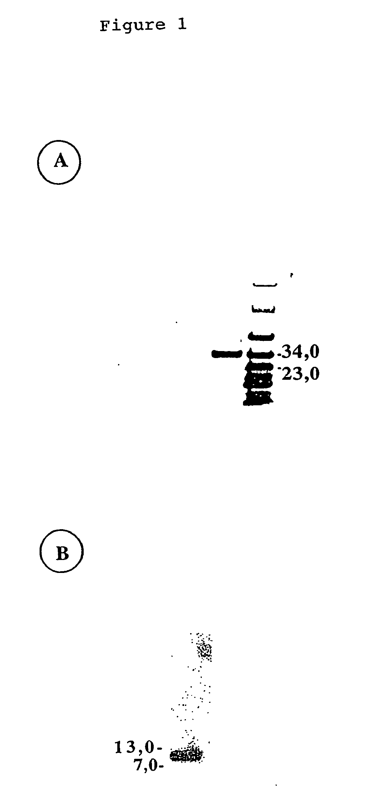

- La figure 1 représente : En a, la visualisation de la protéine de fusion (glutathion-S transferase-AMEP) en SDS-PAGE après purification. Une seule bande est visible après coloration en bleu de Coomassie à 36 Kda. En b, un Western blot de l'AMEP purifié. Visualisation d'une seule bande à 10 Kda par un antisérum anti-disintégrine fait chez le lapin.

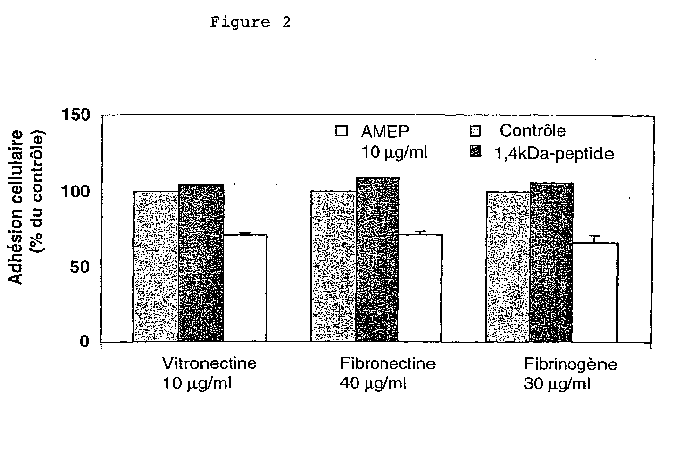

- La figure 2 montre l'effet de l'AMEP sur l'adhésion des CPAE au fibrinogène (30 ug/ml), à la vitronectine (10 µg/ml) et à la fibronectine (40 µg/ml). Les cellules ont été prétraitées pendant 24h avec l'AMEP ou le fragment de 14 acides aminées avant le test d'adhésion (description dans Matériels et méthodes). Les expériences sont réalisées en triple et répétées trois fois. Les résultats sont exprimés en pourcentage par rapport au contrôle (moyenne± SEM).

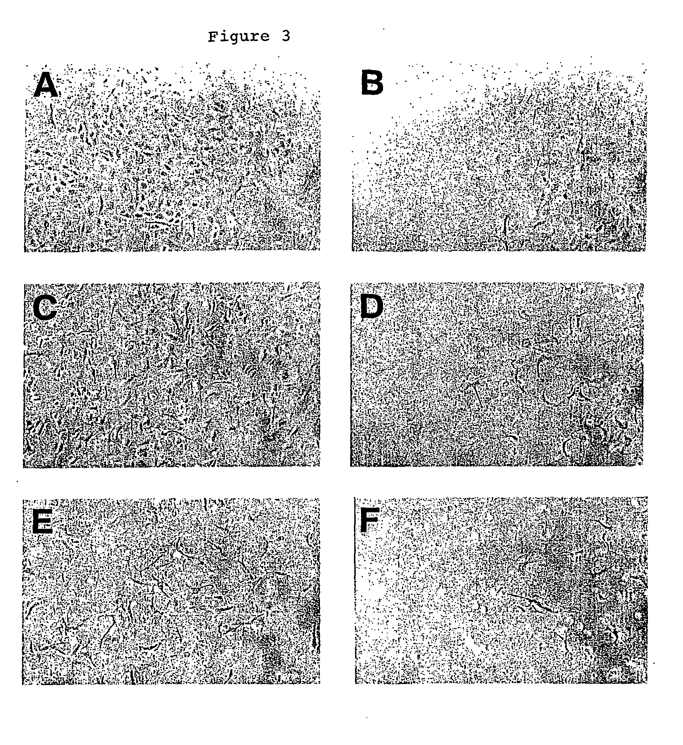

- La figure 3 montre l'effet de l'AMEP sur la morphologie et la migration des cellules endothéliales. Le front de migration est représenté sur les photographies B, D, E respectivement conditions contrôle, AMEP à 5 µg/ml et 10 µg/ml (microscopie à contraste de phase).

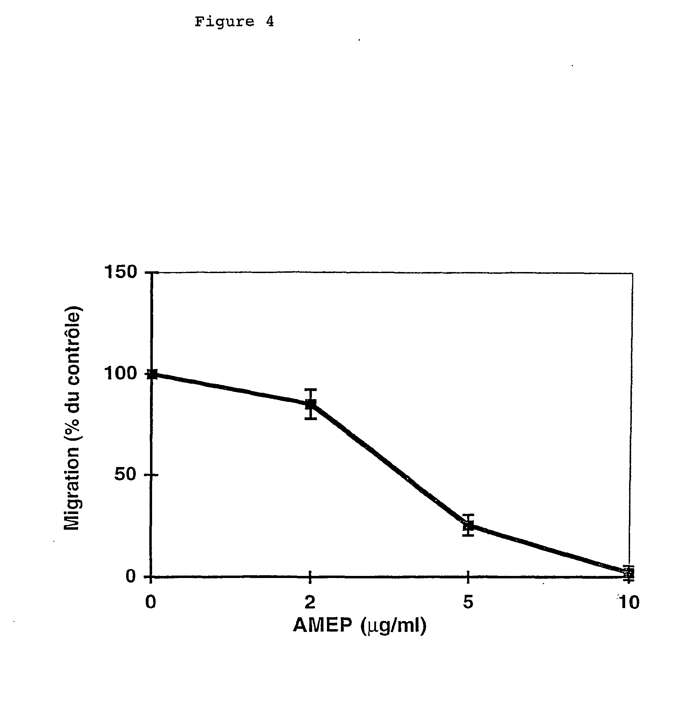

- La figure 4 représente l'inhibition dose dépendante de la migration des CPAE par l'AMEP. La position du front de migration des cellules est mesurée tous les jours pendant une période de 3 jours. Les résultats sont la moyenne de cinq expériences et sont exprimés en pourcentage par rapport au contrôle : moyenne ± SEM.

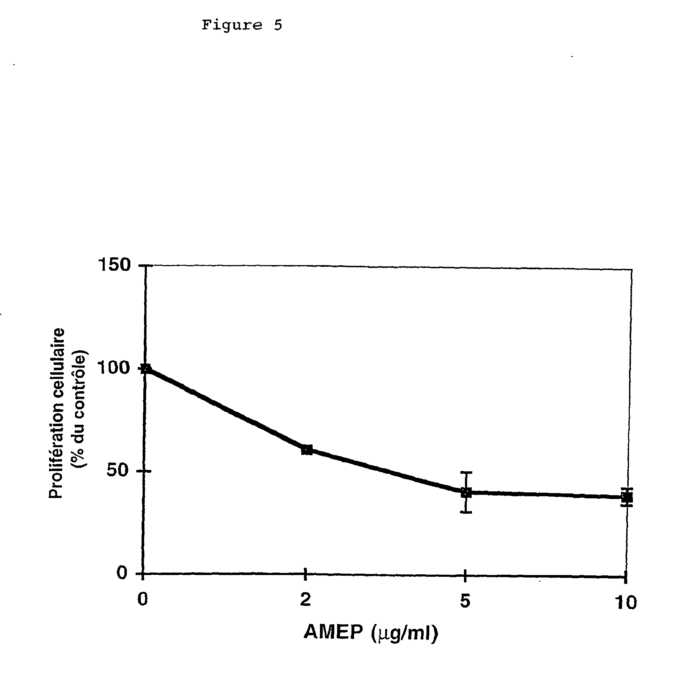

- La figure 5 représente l'inhibition dose dépendante de la prolifération des CPAE par l'AMEP. Les cellules sont cultivées pendant 48h en présence d'AMEP ou d'un fragment de ce domaine, contenant la séquence RGDC, et incubées avec 1 µCi de thymidine tritiée durant 18h. La radioactivité incorporée est alors mesurée. Les résultats sont la moyenne de cinq expériences et sont exprimés en pourcentage par rapport au contrôle : moyenne ± SEM.

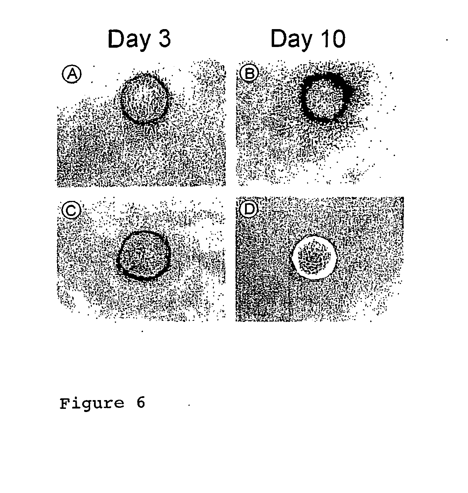

- La figure 6 montre l'effet de l'AMEP sur la formation de structures de type capillaires utilisant les HMEC-1. Des billes de cytodex recouvertes de cellules endothéliales sont incorporées dans un gel de fibrine en absence (photographies A, B) ou en absence d'AMEP (5 µg/ml) (photographies C, D).

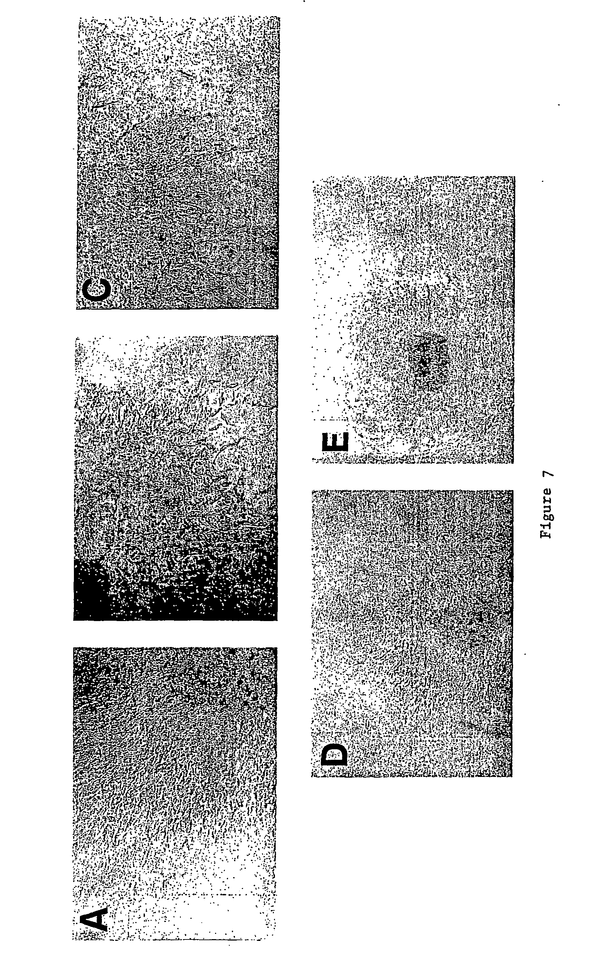

- La figure 7 montre l'effet de l'AMEP sur la formation de capillaires en gel de fibrine utilisant des CPAE en présence de 5 µg/ml (B) ou 10 µg/ml (C, E) d'AMEP par rapport au contrôle (A, D).

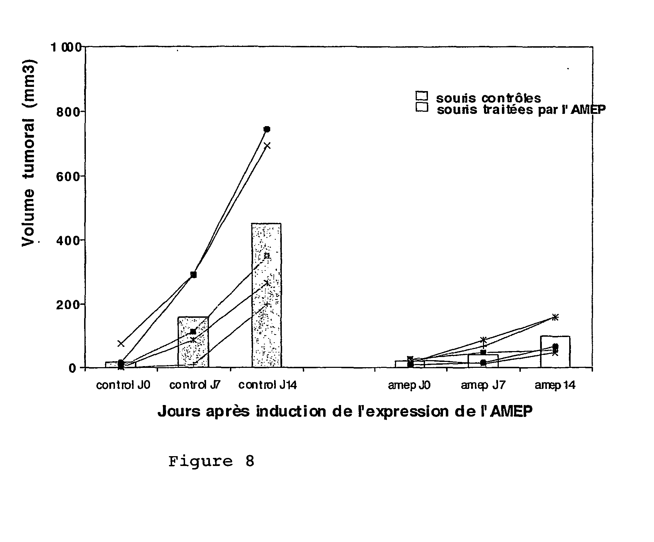

- La figure 8 montre l'inhibition de la croissance tumorale par l'AMEP. Chaque point d'une courbe représente le volume de la tumeur mesuré sur une souris nude. L'expérience présentée implique cinq animaux pour le groupe contrôle ainsi que pour le groupe AMEP, traités pendant 14 jours. Les histogrammes représentent le volume tumoral moyen de 5 souris pour chaque groupe. Cette figure est représentative de 3 expériences distinctes.

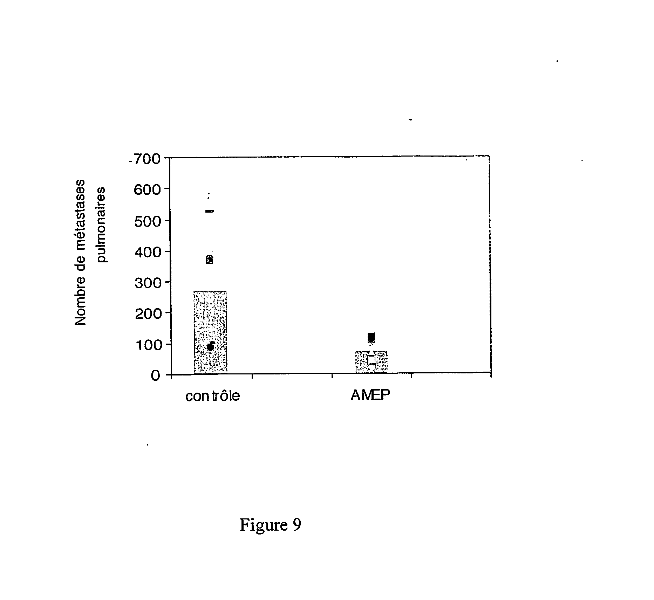

- La figure 9 montre l'inhibition du nombre de métastases pulmonaires par l'AMEP utilisant des cellules de mélanome. Chaque point représente le nombre de métastases comptées dans le poumon d'une souris C57B1/6. L'expérience présentée implique 12 animaux pour chaque groupe. L'histogramme montre la moyenne du nombre de métastases dans le groupe contrôle et traité. Cette expérience est représentative de deux expériences distinctes.

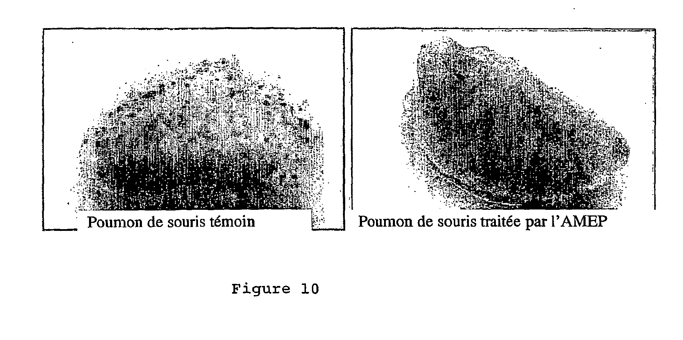

- La figure 10 montre une représentation photographique de l'inhibition du nombre de métastases pulmonaires (taches noires) d'un poumon de souris C57B1/6 traitées par l'AMEP en comparaison avec un poumon de souris contrôle.»

La description ci-après utilise des techniques de biologie moléculaire conventionnelles décrites dans la littérature, comme par exemple : Sambrook, Fritsch et Maniatis, Molecular Cloning; a laboratory Manual, Second Edition (1989) Cold Spring Harbor Laboratory Press, Cold Spring Harbor, New York (Sambrook et coll., 1989); DNA Cloning: A Practical Approch, Volume I et II (D.N. Glover ed. 1985); B. Perdal, A Practical Guide to molecular Cloning (1984) ; F.M. Ausubel et coll. (eds.) Current Protocols in Molecular Biology, John Wiley and Sons, Inc. (1994).

Ainsi, on entend par acide nucléique, un composé polychimérique comprenant des sous-unités liées covalemment appelé nucléotides. Les acides nucléiques incluent l'acide polyribonucléique (ARN) et l'acide polydesoxyribonucléique (ADN) chacun d'entre eux pouvant être simple brin ou double brin. Les ADN incluent l'ADNc (complémentaire), l'ADN génomique, l'ADN synthétique et l'ADN semisynthétique. La séquence de nucléotides ou d'acides nucléiques qui code pour une protéine est appelée séquence sens. On entend par molécule ADN recombinante, une molécule d'ADN qui a subi une manipulation par des techniques de biologie moléculaire.

On entend par séquence codante d'ADN, une séquence d'ADN double brin qui est transcrite et traduite en polypeptide dans une cellule *in vitro* ou *in vivo* quand elle est sous le contrôle de séquences régulatrices appropriées. L'initiation de la séquence codante est déterminée par un codon initiateur en 5' aminoterminale et la fin de la traduction par un codon stop en 3' carboxyterminale. Un signal de polyadénylation (terminaison de la transcription) sera localisé généralement en 3' de la séquence codante. Les séquences contrôlant la transcription et la traduction sont des séquences ADN régulatrices, tels que les promoteurs, les stimulateurs et de fait permettent l'expression d'une séquence codante dans une cellule hôte.

Une séquence promotrice est une région d'ADN capable de lier l'ARN polymérase dans la cellule et d'initier la transcription de la séquence codante.

Une séquence codante est sous le contrôle de séquences transcriptionnelles et traductionnelles dans la cellule lorsque l'ARN polymérase transcrit la séquence codant en ARNm (messager), qui est ensuite traduite en protéine.

Un plasmide d'expression est une molécule d'ADN double brin circulaire extrachromosomique qui comporte des séquences régulatrices entre lesquelles un gène de structure (segment d'ADN correspondant à la protéine désirée) est inséré. Il se réplique de façon indépendante dans les bactéries.

Un vecteur d'expression est une molécule d'acide nucléique qui comporte au minimum une origine de réplication indépendante et un promoteur inductible et qui peut être introduit spécifiquement dans des cellules hôtes telles que dans des bactéries ou des cellules eucaryotes. Dans ce vecteur, peut être insérée une séquence codante appelée insert correspondant à la protéine ou au peptide désiré.

### I - Méthode.

### 1) Culture cellulaire.

Les cellules CPAE (cellules endothéliales d'artère pulmonaire de veau) ont été données par le Dr J. Badet (laboratoire de Biotechnologie des cellules eucaryotes, Université de Créteil, France). Ces cellules sont cultivées dans un milieu MEM additionné de 20% de sérum de veau foetal (SVF), 2mM de L-glutamine, 100 UI/ml de pénicilline et 100 µg/ml de streptomycine (Gibco, Paisley, UK). Tous les milieux cités ci-après (" milieu complet ") contiennent les mêmes concentrations de pénicilline/streptomycine et de L-glutamine que celui utilisé pour les cellules CPAE. Elles sont utilisées aux passages 12-20. Les cellules HMEC-1 (cellules endothéliales de la microvasculature humaine) ont été données par le Dr Ades (Ceter of disease control and prevention, Atlanta, GA) qui a établie cette lignée cellulaire en transfectant des cellules endothéliales du derme humain avec le gène SV40 et l'antigène grand T. Les cellules HMEC-1 sont cultivées dans du milieu MCDB 131 complet (Sigma, St Louis, MO) additionnées de 10% de SVF, 10 ng/ml d'EGF (collaborative Biomédical Products) et 1 µg/ml d'hydrocortisone (Sigma). Les cellules HUVEC (cellules endothéliales de veine des codons ombilicaux humains) ont été extraites au laboratoire de cordon ombilical selon la méthode décrite par Jaffe et collaborateurs. Les cordons sont soumis à une digestion contrôlée par de la collagénase A à 0,2% (Boehringer Manneheim GmbH, Mannheim, Allemagne). Les cellules primaires sont cultivées en milieu complet M199 supplémenté de 20% de SVF, 75 mM HEPES, 3,7 mM de sodium bicarbonate pH 7,5 et 5 µg/ml de fungizone (Life technologies, France). Les cellules HMVEC-d (cellules endothéliales de la microvasculature du derme, Biowhittaker Europe, Belgique) sont cultivées du passage 4 à 6 dans du milieu complet fourni par le fabricant (EGM-2MV) supplémenté de 10% de SVF. Les cellules C51 (cancer du colon murin) sont cultivées en milieu complet RPMI + 10% de SVF. Les cellules 3T3 (fibroblastes tumoraux murins) sont cultivées en milieu complet DNEM + 10%, les cellules MDA MB 231 (cellules de cancer mammaire humaines) utilisent du milieu complet DMEM additionné de 10% de SVF ainsi que les cellules fibroblastiques cancéreuses 3T3. Les cellules B16F10 (cellules de mélanome murin) sont cultivées en milieu DMEM + 10% SVF et 1.5g/l de sodium bicarbonate.

Les anticorps anti-disintégrine sont obtenus après inoculation chez le lapin d'un fragment d'AMEP, selon la méthode décrite par Herren et coll. (Néosystème, France). Ce fragment est constitué de 12 acides aminés et contient la séquence RGDC; son poids moléculaire est de 1,4 KDa.

### 2) Construction et synthèse de l'AMEP humaine dans un système bactérien.

Une protéine de fusion de l'AMEP avec la glutathion S-transferase (GST) a été préparée. Pour ce faire, le fragment d'ADNc de 276 nucléotides de SEQ ID NO.1 qui code pour l'AMEP (Met-420 à Glu-511) a été amplifié par PCR *(polymerase chain reaction).* Cet ADNc a été sous-cloné dans le plasmide pGEX-6P au niveau du site BamH1 (Amersham Pharmacia Biotech). La synthèse de la protéine de fusion GST-AMEP a été induite dans des bactéries Escherichia Coli DH5oparde l'isopropyl-1-thio-ß-D-galactopyranoside (1mM) telle que décrite par Smith et Johnson, 1988. Brièvement, après lyse des bactéries avec 1% de Triton X100 suivit d'une sonication, la GST-Disintegrine a été purifiée sur chromatographie d'affinité utilisant du Glutathion-agarose et éluée avec du Glutathion réduit (5 mM Tris, HCL (pH8,0) contenant 5mM de glutathion réduit (Sigma) (pH 7,5 final, préparé extemporairement). Une seule bande correspondant au poids moléculaire de cette protéine de fusion (36Kda) a été détectée en SDS-PAGE (Figure la).

Afin de tester l'activité du peptide correspondant à l'AMEP sur différents modèles d'angiogénèse in vitro, nous avons clivé l'AMEP de la GST par une protéase spécifique: la « PreScision™ protease », qui est elle-même une protéine couplée à une GST (Amersham, Buckinghamshire), selon les instructions d'Amersham.

L'AMEP est ensuite purifié par chromatographie d'affinité utilisant du Glutathion-agarose sur colonne selon les instructions de Sigma. L'AMEP est constitué de 91 acides aminés et sa masse moléculaire estimée est de 9,7 KDa.

La pureté de l'AMEP a été analysée par western blot (figure 1b) et par chromatographie liquide de haute performance (HPLC). La concentration protéique est déterminée par test BCA (Pierce,Perbio, Science, France).

### 3) Western blot.

Cent microgrammes d'AMEP purifiée ont été déposés sur un gel d'électrophorèse constitué de polyacrylamide-SDS 12% et transférés sur une membrane de nitrocellulose (Schleicher et Schuell). La membrane est saturée pendant 1h avec un tampon TBS (tris buffer solution: Tris 10 mM pH7,5; NaCl 200 mM)/Tween (0,05%) contenant 10% de lait puis incubée avec un sérum polyclonale de lapin à la dilution 1:1000 dirigé contre l'AMEP (Néosystem, France) ou avec un anticorps anti-GST (Amersham). Après cinq lavages en TBS/Tween, la membrane est incubée pendant 1h avec un anticorps secondaire approprié couplé à la peroxydase à la dilution de 1:2000 selon les instructions du fabricant (DAKO). La membrane est lavée cinq fois dans le même tampon de rinçage et la détection du signal est réalisée selon le procédé de chemoluminescence ECL (Amersham).

### 4) Adhésion au fibrinogène, à la vitronectine et à la fibronectine.

- Première technique : incubation ponctuelle des cellules endothéliales avec l'AMEP au cours du test.
   Les CPAE sont décollées des boîtes de culture par incubation avec de l'EDTA 1,5 mM et resuspendu à raison de 5x10⁵ cellules/ml dans du tampon d'adhésion (NaCl 140 mM, Hepes 10 mM, Glucose 5 mM, Kcl 5,4 mM, CaCl2 2 mM, MgCl2 1 mM, MnCl2 1 mM, pH 7,4). Puis, 100 µl de suspension cellulaire sont mis en présence d'AMEP (5 µg/ml) à température ambiante puis introduits dans des puits d'une plaque 96 puits (Greiner, D.Dutcher) préalablement incubés une nuit à 4°C avec 50 µl de fibrinogène purifié (40 µg/ml dans du PBS; Kabi), 10 µg/ml de vitronectine (Sigma); 30 µg/ml de fibronectine (Sigma) ou 1% de BSA (albumine de sérum bovin) comme contrôle négatif. Après 20 min d'incubation des cellules CPAE à 37°C, la plaque est lavée deux fois avec 200 µl de tampon d'adhésion et les sites non spécifiques sont saturés avec du PBS additionné de 1% de BSA pendant 1heure à 37°C. Par la suite la plaque est lavée à nouveau deux fois avec du tampon d'adhésion contenant 1% de BSA. Les cellules non adhérentes sont éliminées par lavage des puits trois fois avec 200 µl de tampon d'adhésion plus 1% de BSA. La mesure de l'activité phosphatase des cellules permet de quantifier les cellules adhérentes. Brièvement, 100 µl de paranitrophénolphosphate (Sigma) à 3 mg/ml dans du tampon acétate pH 5,5 contenant 0,1% de triton X100 est ajouté dans les puits et incubé pendant 2h à 37°C. La réaction est stoppée par l'ajout de NaOH 1N.La libération de paranitrophenol, qui indique le nombre de cellules adhérentes, est mesurée après lecture de l'absorbance à 405 nm par un lecteur ELISA (Titertek Twinreader). Chaque expérience est réalisée trois fois.
- Deuxième technique : les cellules CPAE sont cultivées pendant 24h en présence de l'AMEP à la concentration finale de 5 µg/ml avant d'être décollées. La suite du protocole est identique à celle décrite précédemment à ce détail près qu'aucune autre incubation des cellules avec l'AMEP n'a pas lieu par la suite.

### 5) Modèle de migration des cellules endothéliales.

Le modèle de migration est réalisé dans des plaques de 24 puits. De l'agarose 1,5% est dissout dans du milieu de culture afin de former un gel dans les puits. La moitié d'un cylindre d'agarose est alors insérée dans un puit. Les cellules CPAE sont ajoutées dans l'espace laissé libre du puit et cultivées jusqu'à leur confluence. Le morceau d'agarose est enlevé afin de permettre aux cellules de migrer et l'AMEP est ajouté, aux concentrations désirées, dans le milieu de culture. Puis, un papier millimétré transparent est placé sous la boîte afin de déterminer la vitesse de migration des cellules à l'aide d'un micromètre oculaire sous microscope inversé. Les expériences faites en double sont répétées trois fois. Les résultats sont exprimés en pourcentage par rapport au contrôle

### 6) Modèle de prolifération cellulaire.

Les cellules sont cultivées à raison de 20 000 cellules par puits (plaque de 96 puits: Greiner) dans du milieu complet. Après 24h, les cellules sont cultivées en milieu contenant une concentration moitié moindre que celle correspondant au milieu complet afin d'induire les cellules en phase G0/G1 de prolifération pour 24h supplémentaire. Puis, les cellules sont cultivées pendant 30 h avec du milieu complet en présence ou non de l'AMEP. De la thymidine tritiée (1 µCi par puit) est alors ajoutée aux cellules et incubée pendant 18h. L'incorporation de thymidine tritiée par les cellules est quantifiée grâce à un papier filtre selon le protocole décrit pour l'utilisation d'un Skatron (Skatron, Lier, Norvège), la radioactivité est alors déterminée par un comptage après ajout de liquide à scintillation. Les résultats sont exprimés en pourcentage par rapport au contrôle.

### 7) Analyse de l'apoptose et du cycle cellulaire (utilisation de Hoechst 33342), et quantification de l'apoptose précoce (utilisation de l'Annexine V).

- Technique de coloration vitale spécifique de l'ADN avec le Hoechst 33342 (Sigma).
   Les cellules endothéliales (CPAE) sont trypsinées et la suspension cellulaire est ajustée à 1.10⁶ cellules/ml. Le colorant Hoechst 33342 est ajouté à 20 µg/ml et les cellules incubées trois minutes à température ambiante sous agitation. Le pourcentage de cellules apoptotiques est analysé par un cytomètre de flux (FACS). Les cellules sont ensuite incubées pendant 30 minutes à l'obscurité (37°C) et l'analyse du cycle cellulaire est effectuée.
- Technique utilisant l'annexine V (R et D system): Un culot de 1.10⁶ cellules endothéliales (CPAE) est resuspendu dans 1 ml de tampon de réaction (100 µl de tampon de liaison 10 x (100 mM Hepes/NaOH pH 7,4, 1,5 M NaCl, 50 mM KCl, 10 mM MgCl2, 18 mM CaC12), 100 µl de iodure de propidium (concentration initiale 50 µg/ml), 10 µl d'Annexine V-FITC et 790 µl d'eau désionisée. La suspension est incubée 15 minutes dans l'obscurité à température ambiante. Le pourcentage de cellules apoptotiques est analysé par cytomètre de flux.

### 8) Formation de structures de type capillaire dans deux modèles d'angiogénèse en gel de fibrine.

L'un des modèles utilise des agrégats de cellules CPAE et est utilisé selon la méthode décrite par Pepper et coll.1991. Brièvement 10 000 CPAE sont agrégées pendant 24h sur de l'agarose 2% en plaque de 96 puits. Trois agrégats sont prélevés et incorporés dans un gel de fibrine: du fibrinogène purifié (3mg/ml; Kabi) est dialysé contre du milieu MEM puis mélangé avec 20% de SVF, 1% de L-glutamine, 1% de penicilline/streptomycine,2 µM d'aprotinine et de l'AMEP à la concentration désirée. De la thrombine humaine (1 UI/ml; Sigma) est alors adjoint afin d'obtenir un gel de fibrine à la surface duquel du milieu complet additionné d'aprotinine (2 µM) est ajouté, ce milieu est changé tous les trois jours. La formation de structures de type capillaires peut être observée à partir de 24h de culture. Ces capillaires sont photographiés sous microscope inversé et la taille de ces structures est mesurée sur les photos. L'analyse statistique (Méthode Mann-Whitney) permet de déterminer si la taille de ces structures est différente.

Le deuxième modèle utilise des billes de l'ordre de 150 µm selon la technique décrite par Nehl et coll.. Les cellules utilisées sont les HMEC-1 dans la mesure ou les CPAE ne sont pas utilisables dans ce modèle. Les HMEC-1 ne peuvent, par ailleurs, être employées dans le modèle décrit précédemment. Les cellules HMEC-1 adhérent aux billes Cytodex 3 (Sigma) lorsque les cellules sont incubées avec les billes dans du milieu complet pendant 4h à 37°C. Les billes sont alors resuspendues dans un large volume de milieu complet de façon à avoir 30 cellules/billes et agitées pendant 5 min toutes les 30 min à 30 rpm/min durant 12h, suivit d'une culture en continue à la même vitesse pendant 4 jours. Lorsque toutes la surface des billes est recouverte de cellules, les billes sont centrifugées à 800 g pendant 5 min de façon à les concentrer et incorporer dans un gel de fibrine identique à celui décrit pour le précédent modèle, de même que pour les méthodes de quantification et d'analyses de résultats. Contrairement au modèle utilisant les CPAE, les structures de types capillaires apparaissent seulement après 3 jours de culture à 37°C.

### 9) Préparation des plasmides pour l'électrotransfert.

Le cDNA de l'AMEP est sous cloné au site Eco RV du vecteur pBi (Clonetech, Palo Alto, CA USA). L'expression du gène d'intérêt dans ce vecteur est sous la dépendance d'un promoteur répondant à la tétracycline dans un système d'expression impliquant le gène eucaryote Tet-On. Le vecteur Tet-On exprime le transactivateur rtTA (activateur transcriptionnel tétracycline reverse) et le vecteur TettTS exprime le "silencer" tTS (silencer transcriptionnel tétracycline). Les purifications des plasmides sont effectuées de telle façon qu'aucune endotoxine ne soit présente (Maxi endo free Kit, Quiagen). L'ADN plasmidique purifié est solubilisé dans du NaCl 0,9% stérile, dépourvu d'endotoxine à la concentration voulue.

### 10) Electrotransfert du gène codant pour l'AMEP humaine dans le muscle de souris nude et C57B1/6.

20µg de plasmide pBi-AMEP, 10 µg de plasmide Tet-off et 20µg de plasmide tet-on sont solubilisés dans 30µl NaCl 0,9% stérile et injectés dans le muscle *tibia cranial* de souris nude ou C57B1/6 âgées de 8 semaines et préalablement anesthésiées par inoculation intrapéritonéale de pentobarbital comme décrit par Mir et coll. (Mir et coll., 1999). Brièvement, 8 chocs électriques de 200V/cm sont appliqués pendant 20 ms à une fréquence de 1 Hz, grâce à une électrode adaptée à la patte de la souris et contenant deux plaques d'acier. L'électrode est reliée à un électropulsateur PS-15 (Jouan, St Herblain, France). Les mêmes plasmides ne contenant pas le gène de l'AMEP constituent le contrôle négatif.

### 11) Modèle murin athymique de croissance tumorale (cellules MDA MB 231).

Les cellules MDA-MB-231 préalablement cultivées jusqu'à 80% de confluence sont décollées, lavées et resuspendues dans du PBS à raison de 20.10⁶ cellules/ml. Deux cent microlitres de suspension cellulaire sont injectés en sous-cutanée dans le dos de souris nude de 8 semaines préalablement traitées comme décrit ci-dessus. Les mesures de deux diamètres d'une tumeur permettent de calculer son volume selon la formule mathématique (somme des deux diamètres divisée par 2)³ /0.52. Lorsque les tumeurs atteignent un volume de 18 mm³, de la Doxycycline (analogue stable de la tétracycline) (Sigma-Aldrich, Saint Quentin Fallavier, France) à 200µg/ml est ajoutée dans l'eau de boisson des souris et additionnée de 5% de sucrose, afin d'induire l'expression de l'AMEP dans les muscles de la souris. La taille des tumeurs est suivie pendant 14 jours après induction.

### 12) Quantification de l'angiogénèse tumorale (immunohistochimie et analyse d'image des tumeurs solides sous cutanées).

Les tissus tumoraux sont fixés dans l'éthanol. Des coupes de 5µm sont préparées en paraffine. La peroxydase endogène est éteinte par 3% H2O2 pendant 10 min, afin de pouvoir utiliser ces coupes en immunohistochimie. Après lavage des coupes à l'eau distillée puis saturation avec du sérum Optimax 1:10 (BioGenex, San Ramon, CA) pendant 10 min, les lames sont incubées pendant une 1 hr avec un anticorps de rat anti-CD31 (molécule d'adhésion des cellules endothéliales) au 1:50. Après deux lavages avec l'Optimax pendant 4 min, les lames sont incubées avec un anticorps polyclonal de chèvre anti-rat couplé à la biotine (1/50), suivi par deux lavages de 4min avec l'Optimax. Les lames sont ensuite traitées avec un substrat chromogénique DAB pendant 10 min, lavées avec de l'eau distillée, contre colorées avec de l'hématoxiline de Mayer et montée en Pertex. Toutes les lames sont immunomarquées et contre colorées le même jour ce qui garantit une intensité de marquage standardisée.

Pour chaque animal, un échantillon histologique représentatif des coupes marquées au CD31sont soumis à l'analyse d'image utilisant un microscope AxiophotZeiss (Allemagne), une caméra Sony 3CCD (résolution 768x576 pixels). Le choix d'un grossissement x100 permet la digitalisation de la totalité de l'échantillon. Ces derniers ne prennent en compte que le tissu tumoral, excluant les zones nécrotiques et fibrineuses. Pour chaque échantillon, la totalité de la surface, ou 8 champs contigus si la taille de l'échantillon est trop grande, sont digitalisés. Les images sont analysées par un programme spécifique basé sur le Linux, produisant un index quantitatif de 0 à 255. Les images couleurs digitalisées sont transformées en différents niveaux de gris. Une image théorique composée uniquement de vaisseaux marron-rouges correspondrait à un index 255, tandis qu'une image dépourvue de vaisseaux (marquage bleu entièrement) serait associée à un index 0. Pour chaque pixel de l'image est associée une valeur comprise entre 0 et 255 et la moyenne de ces chiffres est obtenue pour chaque image. L'index final pour chaque animal résulte du calcul de la valeur moyenne de 8 champs contigus.

### 13) Modèle tumoral métastatique syngénique. (métastases pulmonaires, cellules B16F10).

De la Doxycycline (Sigma-Aldrich) à 200µg/ml est ajoutée dans l'eau de boisson des souris C57B1/6 afin d'induire l'expression de l'AMEP dans les muscles de la souris trois jours avant l'injection de cellules de mélanome de souris B16F10. Ces cellules étaient préalablement cultivées jusqu'à 50% de confluence, décollées, lavées et resuspendues dans du PBS à raison de 4.10⁶ cellules/ml. Cent microlitres de suspension cellulaire ont été injectés en intraveineux dans le sinus rétro-orbital des souris. Les souris sont sacrifiées 7 jours après la greffe des cellules, les poumons sont prélevés et sous loupe binoculaire un comptage des métastases pulmonaires de couleur noire est effectué.

### II - Résultats.

### 1) Inhibition de l'adhésion des cellules endothéliales par l'AMEP sur la fibronectine, la vitronectine et le fibrinogène.

Les deux techniques d'adhésion décrites dans les méthodes ont été réalisées en présence de l'AMEP ou d'un fragment de l'AMEP de 1,4 Kda constitué de 12 acides aminés dont la séquence RGDC (Néosystem, France). Ce peptide (" 1,4 kDa-peptide ") a été utilisé afin de déterminer si le mode d'action de l'AMEP diffère d'un peptide RGD contrôle.

Lorsque les cellules endothéliales sont incubées ponctuellement 30 minutes avec le 1,4 kDa-peptide (1 µg/ml) avant de faire le test d'adhésion, une forte réduction de l'adhésion des cellules est observée sur de la vitronectine (49 ± 1,2% d'inhibition) et sur le fibrinogène (50 ± 2,4% d'inhibition). Ce résultat n'est pas surprenant dans la mesure ou ce fragment bloque l'interaction des intégrines alpha v bêta 3 présentes à la surface des cellules endothéliales à ses substrats privilégiés. Dans ces mêmes conditions, aucun effet significatif de l'AMEP sur l'adhésion des cellules endothéliales à ces substrats n'a été détecté à une concentration molaire comparable (10 µg/ml, non montré).

Par contre, lorsque les cellules sont préincubées 24h avec l'AMEP ou avec le 1,4 kDa-peptide à la même concentration molaire que précédemment, les résultats obtenus s'inversent: l'AMEP inhibe l'adhésion des cellules endothéliales sur la vitronectine, la fibronectine et le fibrinogène de 30% pour les trois substrats alors que le 1,4 kDa-peptide n'exerce aucun effet significatif (figure 2).

### 2) Inhibition de la migration des cellules endothéliales par l'AMEP.

La figure 3 montre l'aspect des cellules endothéliales (CPAE) après ajout d'AMEP à 5 µg/ml (C, D) et 10 µg/ml (E, F) comparé au contrôle (absence du domaine: A, B) dans notre modèle de migration. Un très net changement morphologique des cellules est observé en présence de l'AMEP: les cellules endothéliales forment de longs pseudopodes et la cohésion des cellules entre elles est altérée, de nombreuses cellules se décollent (10 µg/ml). Ce phénomène est d'autant plus visible au front de migration des cellules (D, F). L'effet de l'AMEP sur la vitesse de déplacement de ces cellules est dose-dépendant (2 - 10 µg/ml) avec une inhibition complète de la migration des cellules à 10 µg/ml (figure 4) contrairement au 1,4 kDa-peptide (1 µg/ml.) qui n'induit aucun effet inhibiteur.

### 3) Effet de l'AMEP sur la prolifération cellulaire.

Contrairement au fragment de 12 acides aminés (peptide de 1,4 kDa qui n'a aucun effet sur la prolifération des cellules endothéliales quelle que soit la concentration utilisée (1-100 µg/ml, non montré), l'AMEP inhibe fortement leur prolifération (réduction de 40%) dès 2 µg/ml (figure 5). Cet effet est maximum à 5 µg/ml avec 60% d'inhibition de la prolifération (pourcentage identique à 10 µg/ml). A noter que le sérum polyclonale de lapin, préalablement utilisé en western blot (Néosystem, France), dirigé contre l'AMEP inhibe de façon comparable la prolifération de ces cellules (65±0,1%).

Afin d'analyser la spécificité d'action de l'AMEP, nous avons étudié son effet sur la prolifération de cellules endothéliales primaires de la macro- ou microvasculature ainsi que sur des cellules cancéreuses connues pour posséder ou non l'intégrine alpha v bêta 3 à leur surface, l'une des cibles connues de l'AMEP.. Les résultats présentés sur le tableau 1 ci-dessous montrent que l'AMEP inhibe de façon comparable les différents types de cellules endothéliales alors qu'il n'a aucun effet sur la prolifération de cellules cancéreuses de diverses origines (fibroblastiques, mammaires, colon) qui possèdent peu ou pas d'intégrine alphaV béta 3 à leur surface (respectivement MDA MB 231, C51, 3T3). Avantageusement, un effet inhibiteur important de l'AMEP est observé sur la prolifération de cellules exprimant l'intégrine alpha v bêta 3.

**Tableau 1**

| Type cellulaire | Cellules endothéliales | | | Cellules cancéreuses | | | |
|---|---|---|---|---|---|---|---|
| | CPAE bovines | HMVEC-d humaines | HUVEC humaines | 3T3 murines | C51 murines | MDA-MB 231 humaines | B16F10 |
| % d'inhibition de la prolifération cellulaire par l'AMEP (5 µg/ml) | 60,4 ±3,2 | 54,2 ±2,1 | 52,6 ±3,1 | 9,1 ± 2,2 | 0,5 ±1, 1 | 17,5 ±0,5 | 74,3 ±7,0 |

Le tableau 1 rapporte l'effet de l'AMEP sur la prolifération de cellules endothéliales et cancéreuses. Les expériences ont été répétées cinq fois (moyenne ± SEM). Les chiffres indiqués représentent le pourcentage d'inhibition de la prolifération des cellules citées par l'AMEP utilisé à 5 µg /ml par rapport au contrôle (absence d'AMEP) réalisé dans les mêmes conditions.

### 4) Mise en évidence de l'activité proapoptotique de l'AMEP sur les cellules endothéliales.

Deux techniques permettant de déterminer le pourcentage de cellules apoptotiques ont été employées. L'une d'entre elles utilise le Hoechst 33342 qui permet de garder les cellules vivantes et par conséquent de suivre les différentes phases du cycle cellulaire. La technique utilisant l'Annexine-V permet, quant à elle, de déterminer l'apoptose précoce des cellules endothéliales (visualisation des phosphatidyles sérines à leur surface). Les résultats obtenus sont comparables à savoir un pourcentage de cellules apoptotiques multiplié par 3 en présence d'AMEP (Tableau 2 ci-dessous). Par contre, contrairement à la plupart des molécules qui induisent l'apoptose, aucune modification du cycle cellulaire n'a pu être mis en évidence, avec le Hoechst 33342, en présence de l'AMEP (non montré).

**Tableau 2**

| | | Méthode Hoechst 3342 | Méthode Annexine-V |
|---|---|---|---|
| % de cellules apoptotiques | Contrôle | 3,9 ±1,1 | 4,6 ± 0,9 |
| | AMEP 5 µg/ml | 12,6 ± 3,7 | 12,8 ± 2,5 |

Le tableau 2 rapporte le pourcentage de cellules en apoptose. Les analyses en cytométrie de flux sont réalisées selon les deux méthodes décrites dans Matériels et méthodes. Les expériences ont été réalisées trois fois. Moyenne ± SEM.

### 5) L'AMEP inhibe la formation de structure de types capillaires dans deux modèles d'angiogénèse en gel de fibrine.

Les cellules endothéliales de la microvasculature sont plus adaptées pour l'étude de l'angiogénèse, c'est pourquoi nous avons utilisé les HMEC-1. (Nehls et Herrmann, 1995). Ces cellules sont cultivées sur des billes puis incorporées dans un gel de fibrine (Figure 6, contrôle: A, B). L'effet inhibiteur de l'AMEP (5 µg/ml) sur la formation de structures de type capillaire s'observe après trois jours de culture (C) et devient spectaculaire après 10 jours avec une réduction de 90% de la taille des tubes (D). Les études précédentes visant à déterminer les effets de l'AMEP sur les différentes étapes de l'angiogénèse employaient les CPAE comme cellules endothéliales, nous avons donc voulu vérifier l'effet de ce domaine sur un autre modèle d'angiogénèse. En effet, ces cellules ne peuvent être utilisées dans le modèle utilisant des billes car leur morphologie n'est pas adaptée; l'agrégation des CPAE fut l'unique moyen d'étudier l'effet de l'AMEP sur l'angiogénèse in vitro. Les structures de type capillaire apparaissent 24h après l'incorporation des agrégats dans le gel de fibrine. La figure 7 rassemble des photographies prises après 3 jours d'incubation. L'ajout de l'AMEP à 5 µg/ml (B) ou 10 µg/ml (C, E) induit une désorganisation des structures de types capillaires par rapport au contrôle (A,D) aboutissant à une mort des cellules endothéliales (non montré). Dans les conditions contrôle, une augmentation de la longueur des structures est observée jusqu'à 6 jours d'incubation (non montré).

### 6) Inhibition de la croissance tumorale et de l'angiogénèse tumorale par l'AMEP sur des souris nude.

La production d'AMEP dans des souris nude est obtenue après électrotransfert du gène codant pour l'AMEP dans le muscle des souris, suivi d'une induction de son expression par la Doxycycline.

Comme le montre la figure 8, le volume tumoral du groupe AMEP est nettement inférieur à celui du groupe contrôle avec une inhibition constatée qui atteint 78% après 14 jours de traitement. Un pourcentage d'inhibition similaire s'observe après seulement 7 jours de traitement. Une quantification de l'angiogénèse intra-tumorale a été effectuée sur les coupes de ces mêmes tumeurs. Les résultats présentés dans le tableau 3 (ci-dessous) montrent que l'effet inhibiteur puissant de l'AMEP sur la croissance tumorale est corrélé à une inhibition significative du nombre des vaisseaux à l'intérieur des tumeurs traitées par l'AMEP, de 53,4 %.

Ces résultats montrent que l'AMEP agit puissamment dans des modèles *in vivo* de tumeur n'exprimant pas l'intégrine alpha v bêta 3.

Le tableau 3 ci-dessous indique l'index statistique de la vascularisation tumorale, obtenu après digitalisation et analyse d'image par ordinateur d'un échantillon représentatif de coupes de tumeurs provenant de souris exprimant ou non l'AMEP.

**Tableau 3**

| | Groupe contrôle | Groupe Traité par l'AMEP |
|---|---|---|
| Moyenne | 2,96 | 1,38 |
| Variance | 0,28 | 0, 14 |

### 7) Inhibition de la formation de métastases pulmonaires par l'AMEP sur des souris syngéniques.

La production de l'AMEP dans le muscle des souris C57B1/6 est induite par la Doxycycline. Une inhibition exceptionnelle du nombre de métastases pulmonaires de 74,2%, après 7 jours de traitement est observée dans le groupe de souris traitées par l'AMEP, en comparaison avec le groupe contrôle (figures 9,10).

### III - Discussion.

Au cours du processus angiogénique les cellules endothéliales sont activées, elles acquièrent un phénotype angiogénique. Elles possèdent alors à leur surface l'intégrine alpha v bêta 3 et la métargidine (molécules indétectables sur des cellules endothéliales issues de vaisseaux matures) (Herren et coll., 1997).

L'ensemble des résultats obtenus montrent que l'AMEP possède une activité antiangiogénique plus vaste que celle exercée par le 1,4 kDa-peptide. Etant donné que l'AMEP mais également 1,4 kDa-peptide possèdent une séquence RGD impliquée dans la liaison des cellules endothéliales aux intégrines alpha v bêta 3, il est suggéré que l'action de l'AMEP ne se limite pas à un blocage des fonctions de l'intégrine alpha v bêta 3. L'AMEP semble posséder une activité propre qui pourrait être liée à des modifications de signalisation au niveau cellulaire (message qui pourrait transiter par l'intégrine alpha v bêta 3 et/ou par la métargidinee).

L'adhésion des cellules est un phénomène qui intervient dans la migration cellulaire ; cependant nous pensons que l'inhibition de l'adhésion n'est pas le seul mécanisme responsable de l'inhibition de migration des cellules endothéliales : ainsi 10 µg/ml d'AMEP sont suffisants pour bloquer totalement la migration des cellules endothéliales alors que, à cette même concentration, l'effet inhibiteur de l'AMEP sur l'adhésion n'est que partiel.

Cette activité inhibitrice exceptionnelle de l'AMEP sur les étapes clefs de l'angiogénèse est renforcée par son effet antiprolifératif (jusqu 60% d'inhibition de la prolifération des cellules endothéliales). De manière intéressante, l'inhibition de prolifération des cellules endothéliales induite par l'AMEP n'est pas associée à une modification détectable du cycle cellulaire.

Lors de la dernière étape de l'angiogénèse, les cellules s'organisent en tubes qui s'anastomosent pour permettre la formation d'une lumière vasculaire. Nous avons recréé in vitro ce phénomène avec des cellules endothéliales de la microvasculature (HMEC-1) et de la macrovasculature (CPAE) selon deux techniques décrite dans matériels. De façon remarquable, une inhibition totale de la formation de structures de types capillaires est observée dans le modèle utilisant des HMEC-1 en présence d'AMEP. Contrairement à ce que nous avons pu observer jusqu'à maintenant avec d'autres inhibiteurs de l'angiogénèse, l'AMEP induit une désorganisation léthale des tubes de CPAE qui se sont préalablement formés de façon précoce (12h). Les CPAE ont en effet une vitesse de prolifération et de migration bien supérieure aux HMEC-1 (non montré), ce qui permet d'expliquer la dualité d'effet de l'AMEP sur les deux modèles d'angiogénèse.

Ces travaux montrent par ailleurs que l'inhibition de l'angiogénèse tumorale *in vivo* par l'AMEP conduit à une inhibition exceptionnelle de la croissance tumorale, même sur des tumeurs connues pour ne pas exprimer l'intégrine alpha v bêta 3. En outre, un effet anti-métastatique marqué de l'AMEP dans un modèle de métastase pulmonaire est mis en évidence.

Enfin, le traitement des cellules endothéliales avec 10 µg/ml d'AMEP induit une augmentation du nombre de cellules endothéliales mortes (flottantes dans le milieu de culture) observables au microscope à contraste de phase (figure 3). Cependant, l'effet de ce produit sur l'apoptose (phénomène quantifiable sur des cellules encore vivantes) est modeste (augmentation d'un facteur 3). L'AMEP a donc la particularité d'associer un effet antiangiogénique puissant à une induction de la mort cellulaire par un phénomène récemment décrit sous le nom d'anoikis (Frisch, 2000; Zhu et coll., 2001).

L'aspect novateur de l'AMEP réside donc dans sa capacité à inhiber toutes les étapes de l'angiogénèse, y compris la migration et la prolifération des cellules endothéliales ce qui différent des autres molécules découvertes jusqu'à maintenant (cf. l'angiostatine qui inhibe la prolifération des cellules endothéliales O'Reilly et coll., 1994; Wu et coll., 1997, Sim et coll., 1997), ou l'endostatine qui inhibe également leur prolifération ainsi que leur migration mais uniquement lorsque cette dernière est induite par un facteur angiogénique tel que le VEGF ou le bFGF (O'Reilly et coll., 1997, Sim et coll., 2000; Yamaguchi et coll., 1999). De plus, les effets respectifs de l'AMEP sur la migration et l'angiogénèse *in vitro* sont spectaculaires : arrêt total de la mobilité des cellules endothéliales et absence de formation de structures de type capillaire.

De façon inattendue, les effets inhibiteurs puissants de l'AMEP, synthétisé sous forme de protéine recombinante chez la bactérie, sur l'ensemble des expériences décrites *in vitro* sont confirmés par les résultats obtenus *in vivo*, réalisés avec de l'AMEP synthétisée *de novo* chez le mammifère. Avantageusement, l'effet inhibiteur de l'AMEP sur la croissance tumorale dans le modèle athymique est à mettre en relation avec une diminution du nombre de vaisseaux intra-tumoraux, conséquence directe de l'inhibition par l'AMEP, de toutes les étapes de l'angiogénèse *in vitro*. De plus, l'effet anti-invasif de l'AMEP sur la formation de métastases pulmonaires utilisant des cellules de mélanome est corrélé à l'effet anti-prolifératif particulier de l'AMEP sur ces mêmes cellules *in vitro.*

### REFERENCES BIBLIOGRAPHIQUES

Brooks, P.C., Clark, R.A. et Cheresh, D.A. (1994). Requirement of vascular integrin alpha v beta 3 for angiogenesis. Science 264, 569-571.
Eliceiri, B.P., Klemke, R., Stromblad, S. et coll. (1998). Integrin alpha v beta3 requirement for sustained mitogen-activated protein kinase activity during angiogenesis. 140, 1255-1263.
Eliceiri, P.B. et Cheiresh, A. (2000) Role of alpha v integrins during angiogenesis. Cancer J. 6 (suppl: 3), S245-S249.
Folkman, J. (1984).What is the role of endothelial cells in angiogenesis? Lab. Invest. 51, 601-604.
Hammes, H.P., Brownlee, M., Jonczyk, A. et coll., (1996). Subcutaneous injection of a cyclic peptide antagonist of vitronectin receptor-type integrins inhibits retinal neovascularization. Nat Med 2, 529-533.
Frisch SM. (2000) Anoikis.Methods Enzymol. 322, 472-479.
Herren, B., Raines, E. et Ross, R. (1997). Expression of a disintegrin-like protein in cultutred human vascular cells and in vivo. FASEB J., 11, 173-180.
Howard, L., Nelson, K.K., Maciewicz, R.A. et Blobel, C. (1999). Interaction of the metalloprotease disintegrins MDC9 and MDC15 with two SH3 domain-containing proteins, endophilin I and SH3PX1. J. Biol. Chem. 274, 31693-31699.
Jaffer, E.A., Hoyer, L.W. et Nachman, R.L. (1973). Synthesis of antihemophilic factor antigen by cultured human endothelial cells. J. Clin. Invest. 52 2757-2764.
Kang, I-C., Lee, Y-D et Kim, D-S. (1999). A novel disintegrin salmosin inhibits tumor angiogenesis. Cancer research, 59, 3754-3760.
Kostetsky, P.V. et Artemjev, I.V. (2000) Conformational analysis of the biologically active RGD-containing anti-adhesive peptide cyclo(ArgGlyAspPhe-D-val). Biochemistry, 65, 1041-1048.
Klein, S., Glancotti, F., Presta, M., Albelda, S., Buck, C.A. et Rifkin, D.B. (1993). Basic fibroblast growth factor modulates integrin expression in microvascular endothelial cells. Mol. Biol. Cell. 4, 973-982.
Krätschmar, J., Lum, L. et Blobel, C. (1996). Metargidin, a membrane-anchored metalloprotease-disintegrin protein with an RGD integrin binding sequence. J. Biol. Chem. 271, 4593-4596.
Mir, L.M., Bureau, MF., Gehl, J., Rangara, R., Rouy, D., Caillaud, JM., Delaere, P., Branellec, D., Schwartz, B. and Scherman, D. (1999). High-efficiency gene transfer into skeletal muscle mediated by electric pulses. Prc. Natl. Acad. Sci. USA, 96, 4262-4267.
Nehls, V. et Herrmann, R. (1995) The configuration of fibrin clots determines capillary morphogenesis and endothelial cell migration. Micsrovasc. Res. 50, 311-322.
O'Reilly, M.S., Holmgren, L., Shing, Y., Chen, C., Rosentahl, R.A., Moses, M., Lane, W.S., Cao, Y., Sage, E.H. et Folkman, J. (1994). Angiostatin, a novel angiogenesis inhibitor that mediates the suppressionof metastasis by Lewis lung carcinoma. Cell 79, 315-328.
O'Reilly, M.S., Boehm, T., Shing, Y., Fukai, N., Vasios, G., Lane W.S., Flynn, E., Birkhead, J.R., Olsen, B.R. et Folkman, J. (1997). Endostatin: endogenous inhibitor of angiogenesis and tumor growth. Cell 88, 277-285.
Pepper, M.S., Montesano, R., Vassali, J.D. et Orli, L. (1991). Chondrocytes inhibit endothelial sprout formation in vitro: evidence for involvement of a transforming growth factor-beta. J. Cell. Physiol. 146, 170-179.
Primakoff, P. et Myles, D.G. (2000). The ADAM gene familly surface proteins with adhesion and protease activity. TIG 16, 83-87.
Sim, B.K., O'Reilly, M.S., Liang, H., Fortier, A.H., He, W., Madsen, J.W., Lapcevich, R. et Nacy, C.A. (1997). A recombinant human angiostatin protein inhibits experimental primary and metastatic cancer. Cancer Res. 57, 1329-1334.
Smith, D.B. et Johnson K.S. (1988). Single-step purification of polypeptides expressed in Escherichia coli as fusions with glutathion S-transferase. Gene 67, 31-40.
Sim, B.K., MacDonald, N.J. et Gubish, E.R. (2000). Angiostatin and endostatin: endogenous inhibitors of tumor growth. Cancer Metastasis Rev. 19, 181-190.
Wolfsberg, T., Primakoff, P., Myles, D.G. et White J. M. ADAM, (1995), a novel family of membrane proteins containing a disintegrin and metalloprotease domain: multipotential functions in cell-cell and cell-matrix interactions. J. Cell. Biol. 131, 275-278.
Wu, Z., O'Reilly, M.S., Folkman, J. et Shing, Y. (1997). Suppression of tumor growth with recombinant murine angiostatin. Biochem. Biophys. Res. Commun. 236, 651-654.
Yamaguchi, N., Anand-Apte, B., Lee, M., Sasaki, T., Fukai, N., Shapiro, R. Que, I., Lowik, C., Timpl, R. et Olsen, B.R. (1999). Endostatin inhibits VEGF-induced endothelial cell migration and tumor growth independently of zinc binding. EMBO J. 18, 4414-4423.
Yeh, CH., Peng, H-C., Huang, T-F. (1998). Accutin, a new disintegrin, inhibits angiogenesis in vitro and in vivo by actiong as integrin alpha v beta 3 antagonist and inducing apoptosis. Blood, 92, 3268-3276.
Zhang, X-P, Kamata, T., Yokoyama, K., McLaughlin, W., Takada, Y.(1998). Specific interaction of the recombinant disintegrin-like domain of MDC-15 (metargidin, ADAM-15) with integrin alpha v beta 3. J. Biol. Chem. 273, 7345-7350.
Zhu Z, Sanchez-Sweatman O, Huang X, Wiltrout R, Khokha R, Zhao Q, Gorelik E.(2001). Anoikis and metastatic potential of cloudman S91 melanoma cells. Cancer Res. 61, 1707-1716.

## Revendications

1. Molécule d'acide nucléique comprenant ou constituée d'une séquence polynucléotidique codant le domaine disintégrine de la Métargidine ou un dérivé de celui-ci pour une utilisation comme agent anti-angiogénique, anti-invasif ou anti-métastatique, ou ledit dérivé du domaine disintégrine de la Métargidine :
- présente des propriétés anti-angiogéniques, anti-invasives et antimétastatiques ; et
- est constitué d'une séquence modifiée par délétion, addition ou remplacement d'un acide aminé du domaine disintégrine de la Métargidine.

2. Molécule d'acide nucléique selon la revendication 1, **caractérisée en ce que** le domaine disintégrine de la métargidine correspond à la séquence SEQ ID NO : 1.

3. Molécule d'acide nucléique selon les revendications 1 ou 2, **caractérisée en ce qu'**elle est jointe à un vecteur plasmidique d'expression.

4. Cellule transformée par la molécule d'acide nucléique telle que définie dans l'une des revendications 1 à 3, **caractérisée en ce qu'**elle exprime le domaine disintégrine de la métargidine ou un dérivé de celui-ci.

5. Molécule d'acide nucléique selon l'une quelconque des revendications 1 à 4 pour utilisation pour le traitement et/ou la prévention de pathologies choisies parmi les cancers, les processus métastatiques, les maladies inflammatoires, le psoriasis, l'athérosclérose et la dégénérescence maculaire.

## Claims

1. Nucleic acid molecule comprising or consisting of a polynucleotide sequence encoding the disintegrin domain of metargidin or a derivative thereof for use as an anti-angiogenic, anti-invasive or anti-metastatic agent, wherein said derivative of the disintegrin domain of metargidin:
- has anti-angiogenic, anti-invasive and anti-metastatic properties; and
- consists of a sequence modified by deletion, addition or substitution of one amino acid of the disintegrin domain of metargidin.

2. Nucleic acid molecule according to claim 1, **characterised in that** the disintegrin domain of metargidin corresponds to the sequence SEQ ID NO. 1.

3. Nucleic acid molecule according to claims 1 or 2, **characterised in that** it is attached to a plasmid expression vector.

4. Cell transformed by the nucleic acid molecule as defined in one of claims 1 to 3, **characterised in that** it expresses the disintegrin domain of metargidin or a derivative thereof.

5. Nucleic acid molecule according to any one of claims 1 to 4 for use for the treatment and/or prevention of pathological conditions selected from among cancers, metastatic processes, inflammatory diseases, psoriasis, atherosclerosis and macular degeneration.

## Patentansprüche

1. Nucleinsäuremolekül, umfassend eine oder bestehend aus einer Polynucleotidsequenz, die für die Desintegrin-Domäne des Metargidins oder ein Derivat von dieser kodiert, für eine Verwendung als anti-angiogenes, anti-invasives oder antimetastatisches Mittel, bei dem das Derivat der Desintegrin-Domäne des Metargidins:
- anti-angiogene, anti-invasive und anti-metastatische Eigenschaften aufweist; und
- aus einer Sequenz besteht, die durch Deletion, Addition oder Substitution einer Aminosäure der Desintegrin-Domäne des Metargidins modifiziert ist.

2. Nucleinsäuremolekül nach Anspruch 1, **dadurch gekennzeichnet, dass** die Desintegrin-Domäne des Metargidins der Sequenz SEQ ID Nr. 1 entspricht.

3. Nucleinsäuremolekül nach den Ansprüchen 1 oder 2, **dadurch gekennzeichnet, dass** es einem plasmidischen Expressionsvektor beigegeben ist.

4. Zelle, die durch das Nucleinsäuremolekül, wie es in einem der Ansprüche 1 bis 3 definiert ist, transformiert ist, **dadurch gekennzeichnet, dass** sie die Desintegrin-Domäne des Metargidins oder ein Derivat von dieser exprimiert.

5. Nucleinsäuremolekül nach einem der Ansprüche 1 bis 4 zur Verwendung für die Behandlung und/oder Vorbeugung von Krankheiten, die ausgewählt sind aus Krebsen, metastatischen Prozessen, entzündlichen Krankheiten, Psoriasis, Atherosklerose und makularer Degenereszenz.


### SEQUENCE LISTING

<110> Insitut National de la Santé et de la Recherche Médicale (INSERM)
<120> UTILISATION DANS UNE COMPOSITION ANTI-ANGIOGÉNIQUE DU DOMAINE DISINTEGRINE D'ADAMALYSINE
<130> 13723PCT juillet 2002
<140> PCT/FR01/xxxxx
<141> 2002-07-26
<150> FR01/10015
<151> 2001-07-26
<160> 2
<170> PatentIn version 3.1
<210> 1
   <211> 276
   <212> DNA
   <213> Homo sapiens
   <220>
   <221> CDS
   <222> (1)..(276)
   <223> Séquence codante pour le domaine disintégrine de la métargidine
<400> 1
   atg gct gct ttc tgc gga aat atg ttt gtg gag ccg ggc gag cag tgt        48
   Met Ala Ala Phe Cys Gly Asn Met Phe Val Glu Pro Gly Glu Gln Cys
   1               5                   10                  15
   gac tgt ggc ttc ctg gat gac tgc gtc gat ccc tgc tgt gat tct ttg        96
   Asp Cys Gly Phe Leu Asp Asp Cys Val Asp Pro Cys Cys Asp Ser Leu
               20                  25                  30
   acc tgc cag ctg agg cca ggt gca cag tgt gca tct gac gga ccc tgt       144
   Thr Cys Gln Leu Arg Pro Gly Ala Gln Cys Ala Ser Asp Gly Pro Cys
           35                  40                  45
   tgt caa aat tgc cag ctg cgc ccg tct ggc tgg cag tgt cgt cct acc       192
   Cys Gln Asn Cys Gln Leu Arg Pro Ser Gly Trp Gln Cys Arg Pro Thr
       50                  55                  60
   aga ggg gat tgt gac ttg cct gaa ttc tgc cca gga gac agc tcc cag       240
   Arg Gly Asp Cys Asp Leu Pro Glu Phe Cys Pro Gly Asp Ser Ser Gln
   65                  70                  75                  80
   tgt ccc cct gat gtc agc cta ggg gat ggc gag taa                       276
   Cys Pro Pro Asp Val Ser Leu Gly Asp Gly Glu
                   85                  90
<210> 2
   <211> 91
   <212> PRT
   <213> Homo sapiens
   <400> 2
   Met Ala Ala Phe Cys Gly Asn Met Phe Val Glu Pro Gly Glu Gln Cys
   1               5                   10                  15
   Asp Cys Gly Phe Leu Asp Asp Cys Val Asp Pro Cys Cys Asp Ser Leu
               20                  25                  30
   Thr Cys Gln Leu Arg Pro Gly Ala Gln Cys Ala Ser Asp Gly Pro Cys
           35                  40                  45
   Cys Gln Asn Cys Gln Leu Arg Pro Ser Gly Trp Gln Cys Arg Pro Thr
       50                  55                  60
   Arg Gly Asp Cys Asp Leu Pro Glu Phe Cys Pro Gly Asp Ser Ser Gln
   65                  70                  75                  80
   Cys Pro Pro Asp Val Ser Leu Gly Asp Gly Glu
                   85                  90